# EUROPEAN PATENT SPECIFICATION

(11) **EP 1 054 664 B1**
(45) Date of publication and mention of the grant of the patent: **08.08.2012**
(21) Application number: 99903869.8
(22) Date of filing: 11.02.1999
(51) Int. Cl.: A61K 31/185, A61K 31/255, A61P 25/28, A61P 29/00

(54) **METHOD FOR MODULATING MACROPHAGE ACTIVATION**
VERFAHREN ZUR REGULIERUNG VON MACROPHAG-AKTIVIERUNG
METHODE DE MODULATION DE L'ACTIVATION DES MACROPHAGES

(30) Priority: 11.02.1998 US 74295 P; 10.02.1999 US 248396
(43) Date of publication of application: 29.11.2000
(73) Proprietor: BHI Limited Partnership, Laval, QC H7V 4A7 (CA)
(72) Inventor: MORISSETTE, Celine, Montreal, Quebec H4J 2K3 (CA); GERVAIS, Francine, St-Euftache, Quebec J7R 6S9 (CA)
(74) Representative: HOFFMANN EITLE
(86) International application number: PCT/IB1999/000354
(87) International publication number: WO 1999/040909

(56) References cited:
- WO-A-94/22437
- WO-A-96/28187
- WO-A-99/08685
- US-A- 4 386 081
- US-A- 5 276 059
- US-A- 5 389 623
- US-A- 5 463 092
- US-A- 5 869 469
- KISILEVSKY R ET AL: "ARRESTING AMYLOIDOSIS IN VIVO USING SMALL-MOLECULE ANIONIC SULPHONATES OR SULPHATES: IMPLICATIONS FOR ALZHEIMER'S DISEASE" NATURE MEDICINE, vol. 1, no. 2, 1 February 1995, pages 143-148, XP000611547
- T. NAKADA ET AL: "Guanidinoethane sulphate: brain pH alkaline shifter", NEUROCHEMISTRY, vol. 4, no. 8, August 1993 (1993-08), pages 1035-1038,

## Description

### Background of the Invention

Amyloidogenic proteins are a group of proteins which are capable of organizing into extracellular fibrillary protein deposits. These proteins, although different in nature, have a unique set of structural properties: they bind to Congo Red Staining and display an apple green birefringence when observed under polarized light.

Extracellular deposition of Aβ protein in specific regions of the brain is one of the hallmarks of Alzheimer's Disease. Aβ protein is derived from an abnormal proteolytic cleavage of the precursor protein, the βAPP. Once deposited into the brain, it forms senile plaques which have been found in greater numbers in the brains of patients with Alzheimer's Disease. It has also been shown to infiltrate cerebrovascular walls and cause angiopathy. A progressive neuronal cell loss accompanies the deposition of Aβ amyloid fibrils in senile plaques. *In vitro,* Aβ has been shown by several groups to be highly toxic to neurons. La Ferla et al. have recently shown that neuronal cells when exposed *in vitro* to soluble Aβ can become apoptotic. Once internalized, Aβ protein is stabilized and induces DNA fragmentation, which is characteristic of apoptosis. It has been shown that the 25-35 domain of the Aβ protein is responsible for such an excitotoxic activity. These results have brought scientists to consider that not only the organization of Aβ fibrils into senile plaques, which are observed late in the disease, would be detrimental to the host but that even soluble Aβ protein can induce neuronal cell loss earlier in the disease process.

Activated microglia cells have also been observed in brains of patients with Alzheimer's Disease. The activation process of these brain macrophages are thought to be responsible for the presence of inflammatory mediators in brain extracts. These mediators, e.g., inflammatory cytokines, nitric oxide and reactive oxygen intermediates, could play a major role in inducing neuronal cell toxicity. Soluble Aβ protein has recently been shown to be capable of getting internalized by microglial cells and to induce an activation process as determined by production of inflammatory mediators such as NO (Barger et al.). Giulian et al have also shown that this activation process is due to a specific domain of Aβ: the domain of residues 10-16. It is possible that this activation process is due to adherence of the protein (in particular the 10-16 domain of the Aβ protein) to the macrophage cell surface.

WO96/28187 discloses therapeutic compounds and methods for inhibiting amyloid deposition in a subject, whatever its clinical setting. Amyloid deposition is inhibited by the administration to a subject of an effective amount of a therapeutic compound comprising an anionic group and a carrier molecule, or a pharmaceutically acceptable salt thereof, such that an interaction between an amyloidogenic protein and a basement membrane constituent is inhibited.

T. Nakada and I.L. Kwee describe in *NeuroReport* **4**, 1035-1038 (1993) a category of agents, referred to as "*brain pH alkaline shifters*". Using the prototype agent, guanidinoethane sulfonate (GES), the actual alkaline shift in pH was demonstrated in adult mice brain by 31-phosphorus (³¹P) nuclear magnetic resonance (NMR) *in vivo* spectroscopy. The authors state that this alkaline shift was also shown to effectively reduce the extent of brain intracellular lactic acidosis brought about by anoxic insult. These findings are said to support the notion that a pH alkaline shift may protect the brain against the deleterious effects of lactic acidosis. Since higher pH has been shown to significantly reduce beta-amyloid deposition, Nakada and Kwee speculate that alkaline shifters may also have therapeutic potential in Alzheimer's disease.

### Summary of the Invention

It has now been discovered that certain anionic compounds, including sulfonates, are capable of blocking Aβ-induced macrophage activation (or macrophage activation induced by other amyloidogenic proteins or peptides). By interfering with the ability of Aβ to activate macrophages, such compounds can inhibit the inflammatory process, e.g., in the brain of a subject suffering from a disease characterized by Aβ deposition, such as Alzheimer's disease.

The present invention provides the use of 3-aminopropanesulfonic acid, or a pharmaceutically acceptable salt thereof (see Example, *infra*), for the manufacture of a medicament for the treatment of medical indications as specified in appended claim 1. Preferred embodiments of the present invention are specified in the appended dependent claims. Thus, in one embodiment the medicament is for use in a method for inhibiting an inflammatory process (e.g., an inflammatory process due to the presence of, or activation of macrophages by, an amyloidogenic protein or peptide). The method comprises administering to a subject in need thereof (e.g., a subject having amyloid deposition) an effective therapeutic amount of an anionic compound, such that the inflammatory process is inhibited, e.g., by inhibition of macrophage activation by an amyloidogenic protein or peptide, such as Aβ. The subject is a subject having a condition or disease in which Aβ amyloidogenic proteins or peptides are present.

### Brief Description of the Drawings

Figure 1 is a bar graph showing the effect of various conditions on nitric.oxide (NO) production by macrophages in cell culture.
Figure 2 is a bar graph showing the effect of various conditions on Aβ-induced TNFα production by macrophages in cell culture.
Figure 3 is a graph showing the ability of a compound of the invention, 3-aminopropanesulfonic acid, to block or inhibit macrophage activation.

### Detailed Description of the Invention

The invention provides the use of 3-aminopropanesulfonic acid for the manufacture of a medicament intended for therapeutic treatments for subjects suffering from conditions, including inflammation and neuronal cell death in subjects having a condition or disease in which Aβ amyloidgogenic proteins or peptides are present. . By inhibiting the ability of Aβ to induce the activation process of macrophages, neuronal cell loss due to the brain inflammatory status can be slowed or prevented.

As used herein, the terms "inhibiting macrophage activation" or "inhibiting an inflammatory process" refer to decreasing, inhibiting, slowing, ameliorating, or reversing the course or degree of macrophage activation or inflammation, respectively, *in vitro* or in a subject.

In one aspect, administration of the medicament manufactured according to the invention has the effect of inhibiting macrophage activation by an amyloidogenic protein or peptide. The compound to be used in the invention has only a single anionic group. Therefore, the number of anionic groups is not so great as to inhibit traversal of an anatomical barrier, such as a cell membrane, or entry across a physiological barrier, such as the blood-brain barrier, in situations where such properties are desired.

An anionic compound of the invention typically further comprises a counter cation. Cationic groups include positively charged atoms and moieties. Cationic groups include positively charged atoms and moieties. If the cationic group is hydrogen, H⁺, then the compound is considered an acid, e.g., ethanesulfonic acid. If hydrogen is replaced by a metal or its equivalent, the compound is a salt of the acid. Pharmaceutically acceptable salts of the anionic compound are within the scope of the invention. For example, the cationic group can be a pharmaceutically acceptable alkali metal, alkaline earth, higher valency cation (e.g., aluminum salt), polycationic counter ion or ammonium. A preferred pharmaceutically acceptable salt is a sodium salt but other salts are also contemplated within their pharmaceutically acceptable range.

Within the anionic compound, the anionic group is covalently attached to a carrier molecule. The carrier molecule is 3-aminopropane.

As used herein, the term "amino" means -NH₂ . Thus, the term "alkylamino" as used herein means an alkyl group, as defined above, having an amino group attached thereto.

### Pharmaceutically Acceptable Formulations

In the invention, the anionic compound can be administered in a pharmaceutically acceptable formulation. The present invention pertains to any pharmaceutically acceptable formulations, such as synthetic or natural polymers in the form of macromolecular complexes, nanocapsules, microspheres, or beads, and lipid-based formulations including oil-in-water emulsions, micelles, mixed micelles, synthetic membrane vesicles, and resealed erythrocytes.

In one embodiment, the pharmaceutically acceptable formulations comprise a polymeric matrix.

The terms "polymer" or "polymeric" are art-recognized and include a structural framework comprised of repeating monomer units which is capable of delivering an anionic compound, such that treatment of a targeted condition occurs. The terms also include co-polymers and homopolymers, e.g., synthetic or naturally occurring. Linear polymers, branched polymers, and cross-linked polymers are also meant to be included.

For example, polymeric materials suitable for forming the pharmaceutically acceptable formulation employed in the present invention, include naturally derived polymers such as albumin, alginate, cellulose derivatives, collagen, fibrin, gelatin, and polysaccharides, as well as synthetic polymers such as polyesters (PLA, PLGA), polyethylene glycol, poloxomers, polyanhydrides, and pluronics. These polymers are biocompatible with the nervous system, including the central nervous system, they are biodegradable within the central nervous system without producing any toxic byproducts of degradation, and they possess the ability to modify the manner and duration of anionic compound release by manipulating the polymer's kinetic characteristics. As used herein, the term "biodegradable" means that the polymer will degrade over time by the action of enzymes, by hydrolytic action and/or by other similar mechanisms in the body of the subject. As used herein, the term "biocompatible" means that the polymer is compatible with a living tissue or a living organism by not being toxic or injurious and by not causing an immunological rejection.

Polymers can be prepared using methods known in the art (Sandler, S. R.; Karo, W. Polymer Syntheses; Harcourt Brace: Boston, 1994; Shalaby, W.; Ikada, Y.; Langer, R.; Williams, J. Polymers of Biological and Biomedical Significance (ACS Symposium Series 540; American Chemical Society: Washington, DC, 1994). Polymers can be designed to be flexible; the distance between the bioactive side-chains and the length of a linker between the polymer backbone and the group can be controlled. Other suitable polymers and methods for their preparation are described in U.S. Patent Nos. 5,455,044 and 5,576,018, the contents of which are incorporated herein by reference.

The polymeric formulations are preferably formed by dispersion of the anionic compound within liquefied polymer, as described in U.S. Pat. No. 4,883,666, the teachings of which are incorporated herein by reference, or by such methods as bulk polymerization, interfacial polymerization, solution polymerization and ring polymerization as described in Odian G., Principles of Polymerization and ring opening polymerization, 2nd ed., John Wiley & Sons, New York, 1981, the contents of which are incorporated herein by reference. The properties and characteristics of the formulations are controlled by varying such parameters as the reaction temperature, concentrations of polymer and anionic compound, types of solvent used, and reaction times.

In addition to the anionic compound and the pharmaceutically acceptable polymer, the pharmaceutically acceptable formulation of the invention can comprise additional pharmaceutically acceptable carriers and/or excipients. As used herein, "pharmaceutically acceptable carrier" includes any and all solvents, dispersion media, coatings, antibacterial and anti fungal agents, isotonic and absorption delaying agents, and the like that are physiologically compatible. For example, the carrier can be suitable for injection into the cerebrospinal fluid. Excipients include pharmaceutically acceptable stabilizers and disintegrants.

The anionic compound can be encapsulated in one or more pharmaceutically acceptable polymers, to form a microcapsule, microsphere, or microparticle, terms used herein interchangeably. Microcapsules, microspheres, and microparticles are conventionally free-flowing powders consisting of spherical particles of 2 millimeters or less in diameter, usually 500 microns or less in diameter. Particles less than 1 micron are conventionally referred to as nanocapsules, nanoparticles or nanospheres. For the most part, the difference between a microcapsule and a nanocapsule, a microsphere and a nanosphere, or microparticle and nanoparticle is size; generally there is little, if any, difference between the internal structure of the two. In one aspect of the present invention, the mean average diameter is less than about 45 µm, preferably less than 20 µ m, and more preferably between about 0.1 and 10 µm.

In another embodiment, the pharmaceutically acceptable formulations comprise lipid-based formulations. Any of the known lipid-based drug delivery systems can be used in the practice of the invention. For instance, multivesicular liposomes (MVL), multilamellar liposomes (also known as multilamellar vesicles or "MLV"), unilamellar liposomes, including small unilamellar liposomes (also known as unilamellar vesicles or "SUV") and large unilamellar liposomes (also known as large unilamellar vesicles or "LUV"), can all be used so long as a sustained release rate of the encapsulated anionic compound can be established. In one embodiment, the lipid-based formulation can be a multivesicular liposome system. Methods of making controlled release multivesicular liposome drug delivery systems is described in PCT Application Serial Nos. US96/11642, US94/12957 and US94/04490, the contents of which are incorporated herein by reference.

The composition of the synthetic membrane vesicle is usually a combination of phospholipids, usually in combination with steroids, especially cholesterol. Other phospholipids or other lipids may also be used.

Examples of lipids useful in synthetic membrane vesicle production include phosphatidylglycerols, phosphatidylcholines, phosphatidylserines, phosphatidylethanolamines, sphingolipids, cerebrosides, and gangliosides. Preferably phospholipids including egg phosphatidylcholine; dipalmitoylphosphatidylcholine, distearoylphosphatidylcholine, dioleoylphosphatidylcholine, dipalmitoylphosphatidylglycerol, and dioleoylphosphatidylglycerol are used.

In preparing lipid-based vesicles containing an anionic compound, such variables as the efficiency of anionic compound encapsulation, lability of the anionic compound, homogeneity and size of the resulting population of vesicles, anionic compound-to-lipid ratio, permeability, instability of the preparation, and pharmaceutical acceptability of the formulation should be considered (see Szoka, et al., Annual Reviews of Biophysics and Bioengineering, 9:467, 1980; Deamer, et al., in Liposomes, Marcel Dekker, New York, 1983, 27; and Hope, et al., Chem. Phys. Lipids, 40:89, 1986, the contents of which are incorporated herein by reference).

### Administration of the Pharmaceutical Acceptable Formulation

In one embodiment, the anionic compound is suitable to be administered by introduction into the central nervous system of the subject, e.g., into the cerebrospinal fluid of the subject. In certain aspects of the invention, the anionic compound is suitable to be introduced intrathecally, e.g., into a cerebral ventricle, the lumbar area, or the cisterna magna.

The pharmaceutically acceptable formulations can easily be suspended in aqueous vehicles and introduced through conventional hypodermic needles or using infusion pumps. Prior to introduction, the formulations can be sterilized with, preferably, gamma radiation or electron beam sterilization, described in US patent no. 436,742 the contents of which are incorporated herein by reference.

In another embodiment of the invention, the anionic compound formulation is suitable to be administered into a subject intrathecally. As used herein, the term "intrathecal administration" is intended to include delivering an anionic compound formulation directly into the cerebrospinal fluid of a subject, by techniques including lateral cerebroventricular injection through a burrhole or cisternal or lumbar puncture or the like (described in Lazorthes et al. Advances in Drug Delivery Systems and Applications in Neurosurgery, 143-192 and Omaya et al., Cancer Drug Delivery, 1: 169-179, the contents of which are incorporated herein by reference). The term "lumbar region" is intended to include the area between the third and fourth lumbar (lower back) vertebrae. The term "cisterna magna" is intended to include the area where the skull ends and the spinal cord begins at the back of the head. The term "cerebral ventricle" is intended to include the cavities in the brain that are continuous with the central canal of the spinal cord. Administration of an anionic compound to any of the above mentioned sites can be achieved by direct injection of the anionic compound formulation or by the use of infusion pumps. For injection, the anionic compound formulation of the invention can be formulated in liquid solutions, preferably in physiologically compatible buffers such as Hank's solution or Ringer's solution. In addition, the anionic compound formulation may be formulated in solid form and re-dissolved or suspended immediately prior to use. Lyophilized forms are also included. The injection can be, for example, in the form of a bolus injection or continuous infusion (e.g., using infusion pumps) of the anionic compound formulation.

### Duration and Levels of Administration

In another embodiment of the invention, the pharmaceutically acceptable formulation provides sustained delivery, e.g., "slow release" of the anionic compound to a subject for at least one, two, three, or four weeks after the pharmaceutically acceptable formulation is administered to the subject.

As used herein, the term "sustained delivery" is intended to include continual delivery of an anionic compound *in vivo* over a period of time following administration, preferably at least several days, a week or several weeks. Sustained delivery of the anionic compound can be demonstrated by, for example, the continued therapeutic effect of the anionic compound over time (*e.g*., sustained delivery of the anionic compound can be demonstrated by continued inhibition of neuronal cell death over time). Alternatively, sustained delivery of the anionic compound may be demonstrated by detecting the presence of the anionic compound *in vivo* over time.

In one embodiment, the pharmaceutically acceptable formulation provides sustained delivery of the anionic compound to a subject for less than 30 days after the anionic compound is administered to the subject. For example, the pharmaceutically acceptable formulation, e.g., "slow release" formulation, can provide sustained delivery of the anionic compound to a subject for one, two, three or four weeks after the anionic compound is administered to the subject. Alternatively, the pharmaceutically acceptable formulation may provide sustained delivery of the anionic compound to a subject for more than 30 days after the anionic compound is administered to the subject.

The pharmaceutical formulation of the invention, contains a therapeutically effective amount of the anionic compound. A "therapeutically effective amount" refers to an amount effective, at dosages and for periods of time necessary, to achieve the desired result. A therapeutically effective amount of the anionic compound may vary according to factors such as the disease state, age, and weight of the subject, and the ability of the anionic compound (alone or in combination with one or more other agents) to elicit a desired response in the subject. Dosage regimens may be adjusted to provide the optimum therapeutic response. A therapeutically effective amount is also one in which any toxic or detrimental effects of the anionic compound are outweighed by the therapeutically beneficial effects. A non-limiting range for a therapeutically effective concentration of an anionic compound is 100 mM to 1 mM. It is to be further understood that for any particular subject, specific dosage regimens should be adjusted over time according to the individual need and the professional judgment of the person administering or supervising the administration of the anionic compound and that dosage ranges set forth herein are exemplary only and are not intended to limit the scope or practice of the claimed invention.

The invention involves the use of 3-aminopropanesulfonic acid, or a pharmaceutically acceptable salt thereof (see Example, *infra*)*,* for the manufacture of a medicament for the treatment of medical indications as specified in appended claim 1. Thus, in one embodiment, the medicament is for use in a method for inhibiting an inflammatory process (e.g., an inflammatory process due to the presence of, or activation of macrophages by, an amyloidogenic protein or peptide). The method comprises administering to a subject in need thereof (e.g., a subject having amyloid deposition) an effect therapeutic amount of an anionic compound, such that the inflammatory process is inhibited, e.g., by inhibition of macrophage activation by an amyloidogenic protein or peptide, such as Aβ. The subject is a subject suffering from Alzheimer's disease. The anionic compound is homotaurine (3-aminopropanesulfonic acid) or a salt thereof.

### Example 1 (Reference Example)

Macrophages (Bone-marrow derived macrophages - RAW cells) were incubated in serum free medium with Aβ₁₋₄₀ fibrils (Aβ₁₋₄₀ is a polypeptide corresponding to residues 1-40 of the Aβ protein) (final concentration 2.5 µM) with or without the presence of lipopolysaccharide (LPS) (0.01 µg/ml) as a co-inducer of activation. The macrophages were incubated overnight. Supernatants were harvested and inflammatory cytokines TNFα, IL-6, as well as nitric oxide were measured. TNFα, IL-6 were measured by an ELISA, while NO was measured by Griess Reagent.

Negative controls consisted of cells incubated with LPS or Aβ alone. Positive control consisted of cells incubated with LPS and IFNγ at concentrations known to induce an optimal activation of these cells.

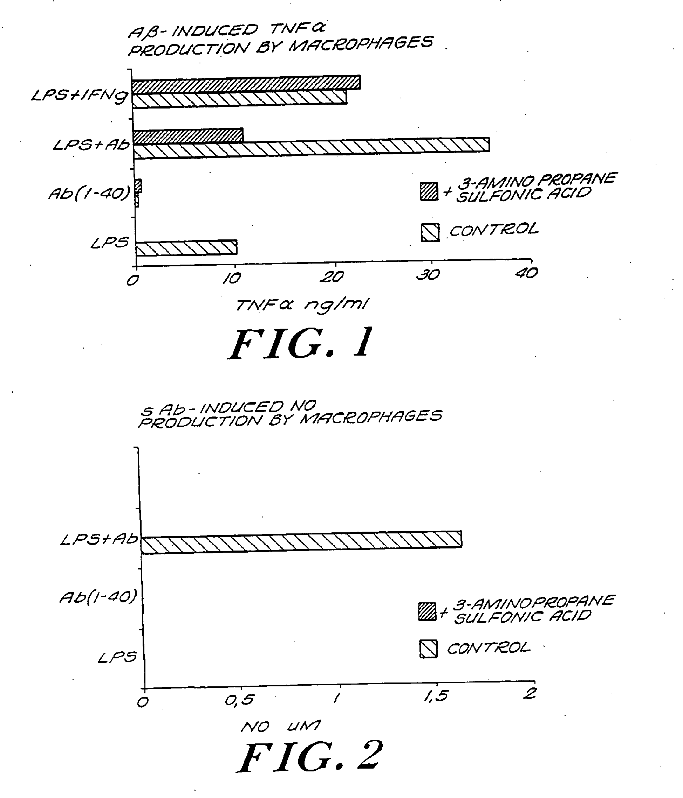

As shown in Figures 1 and 2, 3-aminopropanesulfonic acid (present in solution as the salt form) was found to block about 60% of the Aβ-induced TNFα production, while IL-6 production was not shown to be affected. NO production was also shown to be inhibited by 3-aminopropanesulfonic acid, while this compound did not appear to have any significant effect on the TNFα and NO produced by RAW cells in presence of LPS and IFNγ.

### Example 2 (Reference Example)

The following example demonstrates the ability of compounds of the invention to inhibit Aβ-induced microglia activation.

Human microglia THP-1 cells were primed with LPS (lipopolysaccharide) (0.25 µg/ml) and then incubated with a 5 uM preparation of fibrillary Aβ peptide. Activation was determined by measuring the amount of IL-1β released in the cell culture supernatant. The ability of a compound to block/inhibit the activation process was determined by comparing the amount of cytokine (here, IL-1β) present in the supernatant when cells were incubated with a compound to that obtained in the supernatant of control cells (incubated with Aβ).

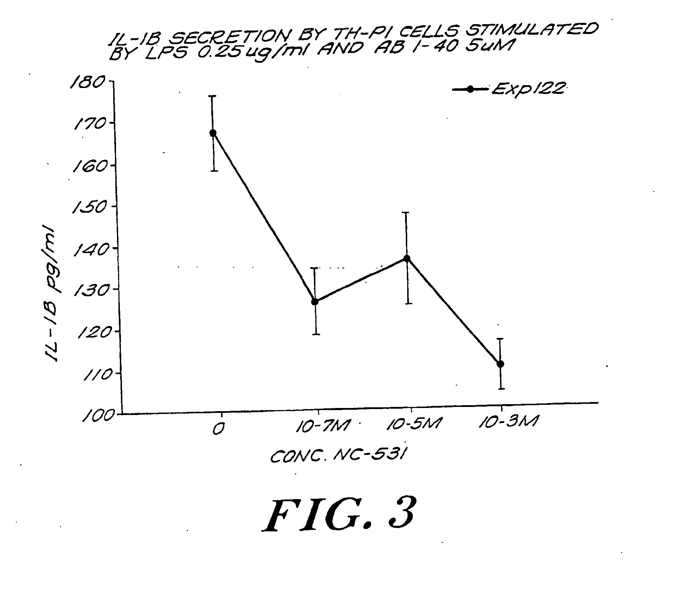

When cells were treated with a sulfonated compound, here, 3-aminopropanesulfonic acid, a significant decrease (shown in FIG. 3) in the amount of IL-1β was seen at concentration of 10⁻⁷M to 10⁻³M, indicating inhibition of microglia.

Those skilled in the art will recognize, or be able to ascertain using no more than routine experimentation, numerous equivalents to the specific procedures described herein. Such equivalents are considered to be within the scope of this invention and are covered by the following claims.

## Claims

1. Compound selected from 3-aminopropanesulfonic acid and pharmaceutically acceptable salts thereof for use in modulating Aβ-induced macrophage activation leading to inflammation, neuronal cell toxicity, neuronal cell death or neuronal cell loss in subjects having a condition or disease in which Aβ amyloidogenic proteins or peptides are present.

2. Compound for use according to Claim 1, wherein the compound is for use in the form of a pharmaceutically acceptable formulation comprising macromolecular complexes, nanocapsules, microspheres or beads.

3. Compound for use according to Claim 1, wherein the compound is for use in the form of a lipid-based formulation selected from the group consisting of oil in water emulsions, micelles, mixed micelles, synthetic membrane vesicles and re-sealed erythrocytes.

4. Compound for use according to Claim 1, wherein the compound is for use in the form of a pharmaceutically acceptable formulation comprising a polymeric matrix.

5. The compound for use according to Claim 4, wherein the polymeric matrix comprises a polymer selected from albumin, alginate, cellulose derivatives, collagen, fibrin, gelatine, polysaccharides, PLA, PLGA, polyethylene glycol, poloxomers, polyanhydrides and pluronics.

6. Compound for use according to Claim 1, wherein the compound is encapsulated in one or more pharmaceutically acceptable polymers to form a microcapsule, microsphere or microparticle.

7. Compound for use according to Claim 3, wherein the lipid-based formulation comprises a combination of phospholipids and steroids.

8. Compound for use according to Claim 3, wherein the lipid-based formulation comprises a lipid selected from phosphatidylglycerols, phosphatidylcholines, phosphatidylserines, phosphatidylethanolamines, sphingolipids, cerebrosides, and gangliosides, preferably phophatidylcholine, dipalmitoylphosphatidylcholine, distearoylphosphatidylcholine, dioleoylphosphatidylcholine, dipalmitoylphosphatidylglycerol, and dioleoylphosphatidylglycerol.

9. Compound for use according to Claim 1, wherein the compound is in the form of a medicament adapted for administration by introduction into the central nervous system.

10. Compound for use according to Claim 9, wherein the medicament is adapted for administration into a cerebral ventricle, the lumbar area, the cisterna magna or the cerebrospinal fluid.

11. The compound for use according to any one of the preceding claims, wherein the compound is for use in the form of a pharmaceutically acceptable formulation providing sustained delivery, wherein sustained delivery means continued delivery of 3-aminopropanesulfonic acid *in vivo* over a period of at least one week.

12. The compound for use according to any one of the preceding claims, wherein the compound is for use in the treatment of soluble Aβ-induced neuronal cell toxicity in subjects suffering from Alzheimer's disease.

13. The compound for use according to any one of the preceding claims 1 to 11, wherein the compound is for use in the treatment of inflammation in subjects suffering from Alzheimer's disease.

14. The compound for use according to any one of the preceding claims 1 to 11, wherein the compound is for use in the treatment of neuronal cell death in subjects suffering from Alzheimer's disease.

15. The compound for use according to any one of the preceding claims 1 to 11, wherein the compound is for use in the treatment of neuronal cell loss in subjects suffering from Alzheimer's disease.

## Patentansprüche

1. Verbindung ausgewählt aus 3-Aminopropansulfonsäure und pharmazeutisch akzeptablen Salzen hiervon zur Verwendung bei der Regulierung der Aß-induzierten Makrophagen-Aktivierung, die bei Patienten mit einer Störung oder Erkrankung, bei der Aβ-amyloidogene Proteine oder Peptide vorliegen, zu Entzündungen, neuronaler Zelltoxizität, neuronalem Zelltod oder neuronalem Zellverlust führt.

2. Verbindung zur Verwendung nach Anspruch 1, wobei die Verbindung zur Verwendung in der Form einer pharmazeutisch akzeptablen Formulierung vorliegt, umfassend makromolekulare Komplexe, Nanokapseln, Mikrosphären oder Beads.

3. Verbindung zur Verwendung nach Anspruch 1, wobei die Verbindung zur Verwendung in Form einer Formulierung auf Lipidbasis, ausgewählt aus der Gruppe bestehend aus Ölin-Wasser-Emulsionen, Mizellen, gemischten Mizellen, synthetischen Membranvesikeln und wiederverschlossenen Erythrozyten, vorliegt.

4. Verbindung zur Verwendung nach Anspruch 1, wobei die Verbindung zur Verwendung in Form einer pharmazeutisch akzeptablen Formulierung vorliegt, umfassend eine Polymermatrix.

5. Verbindung zur Verwendung nach Anspruch 4, wobei die Polymermatrix ein Polymer, ausgewählt aus Albumin, Alginat, Zellulosederivaten, Collagen, Fibrin, Gelatine, Polysacchariden, PLA, PLGA, Polyethylenglykol, Poloxomeren, Polyanhydriden und Pluronics, umfasst.

6. Verbindung zur Verwendung nach Anspruch 1, wobei die Verbindung in einer oder mehreren pharmazeutisch akzeptablen Polymeren eingeschlossen ist, um eine Mikrokapsel, Mikrosphäre oder einen Mikropartikel zu bilden.

7. Verbindung zur Verwendung nach Anspruch 3, wobei die Formulierung auf Lipidbasis eine Kombination von Phospholipiden und Steroiden umfasst.

8. Verbindung zur Verwendung nach Anspruch 3, wobei die Formulierung auf Lipidbasis ein Lipid, ausgewählt aus Phosphatidylglycerinen, Phosphatidylcholinen, Phosphatidylserinen, Phosphatidylethanolaminen, Sphingolipiden, Cerebrosiden und Gangliosiden, bevorzugt Phosphatidylcholin, Dipalmitoylphosphatidylcholin, Distearoylphosphatidylcholin, Dioleoylphosphatidylcholin, Dipalmitoylphosphatidylglycerin und Dioleoylphosphatidylglycerin umfasst.

9. Verbindung zur Verwendung nach Anspruch 1, wobei die Verbindung in Form eines Medikamentes vorliegt, welches zur Verabreichung durch Einbringen in das zentrale Nervensystem geeignet ist.

10. Verbindung zur Verwendung nach Anspruch 9, wobei das Medikament zur Verabreichung in einer Hirnkammer, dem Lendenwirbelbereich, der Cisterna magna oder der cerebrospinalen Flüssigkeit geeignet ist.

11. Verbindung zur Verwendung nach einem der vorhergehenden Ansprüche, wobei die Verbindung zur Verwendung in Form einer pharmazeutisch akzeptablen Formulierung vorliegt, die verzögerte Abgabe bereitstellt, wobei verzögerte Abgabe kontinuierliche Abgabe von 3-Aminopropansulfonsäure in vivo über einen Zeitraum von wenigstens einer Woche bedeutet.

12. Verbindung zur Verwendung nach einem der vorhergehenden Ansprüche, wobei die Verbindung zur Verwendung bei der Behandlung von neuronaler Zelltoxizität, die durch lösliches Aβ induziert wird, bei Patienten dient, die an Morbus Alzheimer leiden.

13. Verbindung zur Verwendung nach einem der vorhergehenden Ansprüche 1 bis 11, wobei die Verbindung zur Verwendung bei der Behandlung von Entzündungen bei Patienten dient, die an Morbus Alzheimer leiden.

14. Verbindung zur Verwendung nach einem der vorhergehenden Ansprüche 1 bis 11, wobei die Verbindung zur Verwendung bei der Behandlung von neuronalem Zelltod bei Patienten dient, die an Morbus Alzheimer leiden.

15. Verbindung zur Verwendung nach einem der vorhergehenden Ansprüche 1 bis 11, wobei die Verbindung zur Verwendung bei der Behandlung von neuronalem Zellverlust bei Patienten dient, die an Morbus Alzheimer leiden.

## Revendications

1. Composé choisi parmi l'acide 3-aminopropanesulfonique et les sels pharmaceutiquement acceptables de celui-ci destiné à une utilisation dans la modulation de l'activation des macrophages induite par l'Aβ conduisant à une inflammation, une toxicité envers les cellules neuronales, la mort des cellules neuronales ou la perte de cellules neuronales chez des sujets présentant un état ou une maladie dans laquelle des protéines ou des peptides amyloïdogènes Aβ sont présents.

2. Composé destiné à une utilisation selon la revendication 1, dans lequel le composé est destiné à une utilisation sous la forme d'une formulation pharmaceutiquement acceptable comprenant des complexes macromoléculaires, des nanocapsules, des microsphères ou des perles.

3. Composé destiné à une utilisation selon la revendication 1, dans lequel le composé est destiné à une utilisation sous la forme d'une formulation à base de lipides choisie dans le groupe constitué par les émulsions huile dans eau, les micelles, les micelles mixtes, les vésicules de membranes synthétiques et les érythrocytes rescellés.

4. Composé destiné à une utilisation selon la revendication 1, dans lequel le composé est destiné à une utilisation sous la forme d'une formulation pharmaceutiquement acceptable comprenant une matrice de polymère.

5. Composé destiné à une utilisation selon la revendication 4, dans lequel la matrice de polymère comprend un polymère choisi parmi l'albumine, un alginate, les dérivés de la cellulose, le collagène, la fibrine, la gélatine, les polysaccharides, le PLA, le PLGA, le polyéthylène glycol, les poloxomers, les polyanhydrides et les pluronics.

6. Composé destiné à une utilisation selon la revendication 1, dans lequel le composé est encapsulé dans un ou plusieurs polymères pharmaceutiquement acceptables pour former une microcapsule, une microsphère ou une microparticule.

7. Composé destiné à une utilisation selon la revendication 3, dans lequel la formulation à base de lipides comprend une combinaison de phospholipides et de stéroïdes.

8. Composé destiné à une utilisation selon la revendication 3, dans lequel la formulation à base de lipides comprend un lipide choisi parmi les phosphatidylglycérols, les phosphatidylcholines, les phosphatidylsérines, les phosphatidyléthanolamines, les sphingolipides, les cérébrosides, et les gangliosides, de préférence la phosphatidylcholine, la dipalmitoylphosphatidylcholine, la distéaroylphosphatidylcholine, la dioléoylphosphatidylcholine, le dipalmitoylphosphatidylglycérol, et le dioléoylphosphatidylglycérol.

9. Composé destiné à une utilisation selon la revendication 1, dans lequel le composé est sous la forme d'un médicament adapté à une administration par introduction dans le système nerveux central.

10. Composé destiné à une utilisation selon la revendication 9, dans lequel le médicament est adapté à une administration dans un ventricule cérébral, la zone lombaire, la grande citerne ou le liquide céphalorachidien.

11. Composé destiné à une utilisation selon l'une quelconque des revendications précédentes, dans lequel le composé est destiné à une utilisation sous la forme d'une formulation pharmaceutiquement acceptable fournissant une libération prolongée, dans laquelle la libération prolongée désigne une délivrance continue d'acide 3-aminopropanesulfonique *in vivo* sur une période d'au moins une semaine.

12. Composé destiné à une utilisation selon l'une quelconque des revendications précédentes, dans lequel le composé est destiné à une utilisation dans le traitement d'une toxicité envers les cellules neuronales induite par l'Aβ soluble chez des sujets souffrant de la maladie d'Alzheimer.

13. Composé destiné à une utilisation selon l'une quelconque des revendications 1 à 11 précédentes, dans lequel le composé est destiné à une utilisation dans le traitement de l'inflammation chez des sujets souffrant de la maladie d'Alzheimer.

14. Composé destiné à une utilisation selon l'une quelconque des revendications 1 à 11 précédentes, dans lequel le composé est destiné à une utilisation dans le traitement de la mort des cellules neuronales chez des sujets souffrant de la maladie d'Alzheimer.

15. Composé destiné à une utilisation selon l'une quelconque des revendications 1 à 11 précédentes, dans lequel le composé est destiné à une utilisation dans le traitement de la perte de cellules neuronales chez des sujets souffrant de la maladie d'Alzheimer.
